# EUROPEAN PATENT APPLICATION

(11) **EP 4 480 432 A2**
(43) Date of publication of application: **25.12.2024**
(21) Application number: 24205364.3
(22) Date of filing: 11.08.2020
(51) Int. Cl.: A61B 18/14

(54) **SYSTEMS FOR A DISSECTION TOOL**

(30) Priority: 15.08.2019 US 201962887230 P
(62) Divisional of application: 20761072.6
(71) Applicant: Boston Scientific Scimed Inc., Maple Grove, Minnesota 55311 (US)
(72) Inventor: The designation of the inventor has not yet been filed
(74) Representative: Vossius & Partner Patentanwälte Rechtsanwälte mbB

(57) **Abstract**

A medical device comprises a body having a proximal end with a proximal opening. The body defines a channel from the proximal opening to a distal opening configured to emit a fluid jet along a longitudinal axis of the body. The body further includes a distal wall extending in a direction transverse to the longitudinal axis and having an inner surface which faces the distal opening to receive the fluid jet. The body defines a space between the distal wall and the distal opening. The distal wall includes a protrusion configured to engage tissue. The body receives a fluid at the proximal opening and further comprises a valve and a spring configured to maintain the fluid within the body until a pressure of the fluid is above a predetermined threshold.

## Description

### CROSS-REFERENCE TO RELATED APPLICATIONS

This application claims the benefit of priority from U.S. Provisional Application No. 62/887,230, filed August 15, 2019, which is incorporated by reference herein in its entirety.

### TECHNICAL FIELD

The present disclosure relates generally to medical devices, including endoscopic devices for tissue resection. In particular, embodiments of this disclosure relate to systems and devices for a fluid powered endoscopic dissection tool.

### BACKGROUND

Tumor resection and other tissue treatment is often performed by medical devices (e.g., endoscopic devices) by delivering radio frequency (RF) energy to destroy tissue. For malignant tumor resection, it may be desirable to preserve tissue architecture to confirm accurate diagnosis and to confirm complete removal and treatment of the tissue. Since tissue architecture may be destroyed during RF energy delivery, there may be a delayed or incomplete medical confirmation of a successful tissue resection. For example, the destroyed tissue architecture may delay or inhibit proper biopsy and classification of the treated tissue. Additionally, RF energy delivery devices may cause postoperative complications and tissue artifact as a result of, for example, delayed tissue effects. Therefore, a need exists for a fast, accurate, and precise method of resection with minimal collateral tissue damage.

### SUMMARY

According to an example, a medical device is provided. The medical device comprises a body that has a proximal end with a proximal opening. The body defines a channel from the proximal opening to a distal opening configured to emit a fluid jet along a longitudinal axis of the body. The body further includes a distal wall surface that has a surface extending in a direction transverse to the longitudinal axis and faces the distal opening to receive the fluid jet. The body defines a space between the distal wall surface and the distal opening. The distal wall includes a protrusion configured to engage tissue.

In another example embodiment, a medical device comprises a tubular member that has a proximal end couplable to a fluid source and a distal end. The medical device has a fluid channel disposed in the tubular member and is configured to deliver fluid from the proximal end of the tubular member through the tubular member to the distal end. The medical device has a nozzle located at the distal end of the tubular member. The nozzle is configured to emit a fluid jet along a longitudinal axis. The distal wall has a surface extending in a direction transverse to the longitudinal axis and faces the nozzle to receive the fluid jet. The wall includes a protrusion configured to engage tissue. The medical device defines a space between the nozzle and the distal wall.

In another embodiment, a method of medical tissue is provided. The method comprises placing a medical device proximate a tissue of interest, engaging the tissue of interest with a protrusion of the medical device to hold the medical device in a position proximate the tissue of interest, and emitting fluid from a distal opening of the medical device towards a distal wall surface of the medical device along a longitudinal axis. The fluid pierces the tissue of interest.

In some example embodiments, the distal opening emits the fluid jet at a pressure to pierce the tissue. The pressure of the fluid jet may be at or below 250 pounds per square inch and the distal opening may have a diameter of approximately 1 millimeter or less. The proximal opening has a diameter that is larger than a diameter of the distal opening. The channel tapers in a cross-sectional size from the distal opening to the proximal opening, and the protrusion comprises one or more pointed tips to engage the tissue.

In additional embodiments, the body further comprises a valve and a spring, the valve being fixedly coupled and disposed proximal to the spring and positioned between the proximal opening and the distal opening of the tubular member. The body may be electrically conductive to deliver radio frequency (RF) energy to the tissue. The RF energy delivered to the body is conductive to the distal wall surface. The body comprises a bottom surface disposed along the longitudinal axis between the proximal opening and the distal wall surface to define a region to engage tissue. The medical device also comprises a flexible tube coupled to the proximal end of the body. The flexible tube has a channel to deliver fluid to the body. The flexible tube includes an electrically conductive tube, wire, cable, or braid for delivery of radio frequency (RF) energy to the body.

It may be understood that both the foregoing general description and the following detailed description are exemplary and explanatory only and are not restrictive of the invention, as claimed. As used herein, the terms "comprises," "comprising," or any other variation thereof, are intended to cover a non-exclusive inclusion, such that a process, method, article, or apparatus that comprises a list of elements does not include only those elements, but may include other elements not expressly listed or inherent to such process, method, article, or apparatus. The term "exemplary" is used in the sense of "example," rather than "ideal." As used herein, the term "proximal" means a direction closer to an operator, and the term "distal" means a direction further from an operator. Although endoscopy is referenced herein, such reference should not be construed as limiting the possible applications of the disclosed tools. For example, the disclosed tools may be used in procedures such as bronchoscopy, ureteroscopey, colonoscopey, or other procedures within the body.

### BRIEF DESCRIPTION OF THE DRAWINGS

The accompanying drawings, which are incorporated in and constitute a part of this specification, illustrate examples of the present disclosure and together with the description, serve to explain the principles of the disclosure.
FIG. 1 is a perspective view of a medical device performing a fluid-powered tissue dissection, according to an embodiment of the disclosure.
FIGs. 2A and 2B show the distal end of the medical device of FIG. 1.
FIG. 3 shows a cross-sectional view of the medical device of FIG. 1.
FIGs. 4A-4C show a cross-sectional view of another embodiment of a medical device.
FIG. 5 shows a cross-sectional view of yet another embodiment of a medical device according to this disclosure and configured to perform dual fluid-powered tissue dissection and an RF energy delivery to tissue.
FIG. 6 shows an example flow chart depicting operations for performing a fluid-powered tissue dissection.

### DETAILED DESCRIPTION

Tissue dissection, and specifically tissue/tumor resection and removal, may benefit from a medical device that implements a fast, accurate, and precise method of resection with minimal collateral tissue damage. Such medical devices may be more effective and advantageous than medical devices that deliver only radio frequency (RF) energy to perform tissue resection, for example, by preserving tissue architecture for confirmation of medical diagnosis and for effective tissue treatment. In some embodiments, fluid may be delivered to perform tissue dissection techniques (e.g., submucosal dissection). Fluid powered systems may be configured to dissect or resect tissue effectively with high precision and low heat. Additionally, fluid powered resection systems may be combined with RF or other energy delivery technologies to provide coagulation and hemostatic function during tissue resection. In one example, fluid powered tissue resection techniques may lead to reduced blood loss during a surgical procedure, and the low temperature helps preserve tissue and vessel architecture. Resection depth can also be controlled with varying fluid pressure applications. Therefore, aspects of the present disclosure are directed to medical devices with fluid powered tissue resection systems.

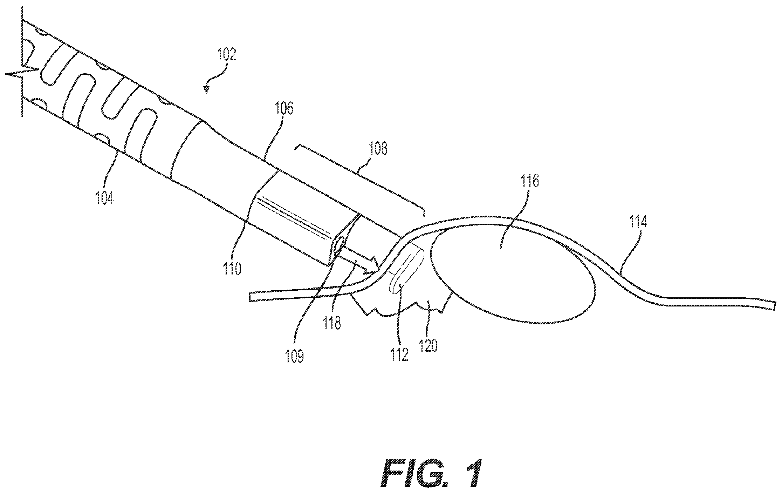

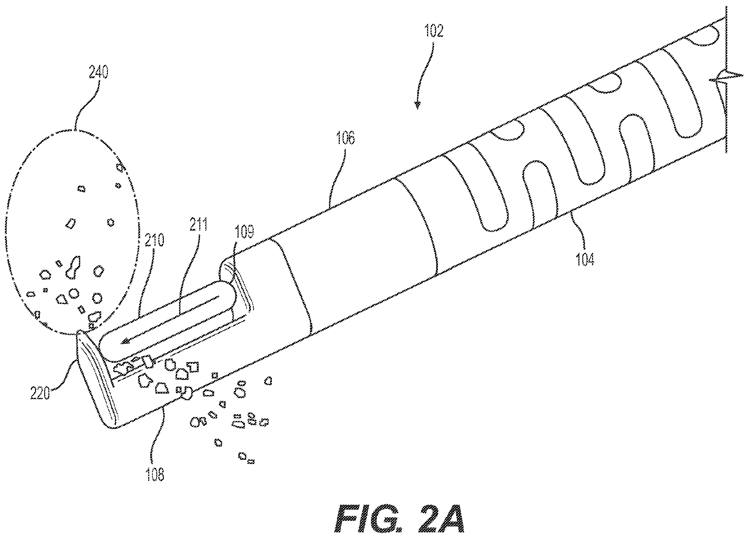

Reference is now made to FIG. 1. FIG. 1 depicts an example medical device 102. The medical device 102 may be, for example, an endoscopic medical device, such as a catheter, used to perform tissue resection methods (e.g., submucosal tissue dissection). The medical device 102 has a tubular member 104 with a proximal end (not shown) and a distal end 106. The tubular member 104 may be any known or contemplated tubular member 104 used for medical procedures, such as endoscopy, and may be, for example, a flexible tubular member with one or more channels or lumens disposed therein and extending between the proximal and distal ends 106 of the tubular member 104 for medical operation. In one example, the tubular member 104 is a catheter, which may be solid, slotted, braided, injection molded, or reflown. As shown in FIG. 1 and FIG. 2A, at least a distal portion of tubular member 104 is slotted to, for example, add flexibility to the tubular member 104. The slotted portion may extend to an unslotted portion at distal end 106, which couples to a body 108 to be described. In one example, the tubular member may be an extruded device. The medical device 102 also has a handle (not shown) connected to the proximal end of the tubular member 104. An operator may use the handle to perform operations of the medical device 102, including those described by the examples herein. The handle may be any known or contemplated handle used for medical procedures, and may include suitable ports and plugs for fluid and/or energy delivery. Additionally, the medical device 102 has a fluid delivery mechanism (not shown) located at the proximal end of the tubular member 104. The fluid delivery mechanism may or may not be part of the handle, and enables fluid to flow from the proximal end of the tubular member 104, via one or more internal channel or lumens, to the distal end 106 and ultimately to a body 108 to perform the fluid powered tissue resection techniques described herein. In one example, the fluid delivery mechanism may include a fluid source that is disposed at the proximal end of the tubular member 104, for example within the handle. In another example, the fluid delivery mechanism may be a mechanism to drive fluid through the tubular member 104 (e.g., a pump) from a remote fluid source.

The medical device 102 also includes a body 108 disposed on the distal end 106 of the tubular member 104. The body 108 has a proximal end 110 and a distal end 112. In one example, the proximal end 110 of the body 108 is configured to interface with/engage the distal end 106 of the tubular member 104. For example, the body 108 may plug into a mating component at the distal end 106 of the tubular member 104. It should be appreciated that any internal working channels and/or lumens may be aligned in the body 108 and the tubular member 104. In other examples, the body 108 may be integrally formed with and permanently fixed to the tubular member 104 (e.g., by being bonded or otherwise adhered or affixed to the tubular member 104). The body 108 has a proximal opening (not shown in FIG. 1) and a distal opening 109.

FIG. 1 also shows a tissue boundary 114. The tissue boundary 114 may be any tissue layer within the human body. FIG. 1 also shows a target tissue at reference numeral 116. In one example, the target tissue 116 may be a tumor located within a gastrointestinal (GI) endothelium, though it should be appreciated that this may be any tissue in the human body. The techniques described herein enable treatment of the target tissue 116, for example, by providing a fluid powered tissue resection method. FIG. 1 shows the distal end 112 of the body 108 embedded below the tissue boundary 114. Reference numeral 118 shows a direction at which fluid may be delivered at a sufficiently high pressure to pierce the tissue boundary 114 and a tissue region 120 where treatment is being applied. Ultimately, the medical device 102 is used to resect the target tissue 116. These systems and methods are described in more detail herein.

Reference is now made to FIG. 2A, which shows the body 108 at the distal end 106 of the tubular member 104 according to one example embodiment. As stated above, the body 108 is configured to interface with the tubular member 104, e.g., by plugging into an opening (not shown in FIG. 1) at the distal end 106 of the tubular member 104. The body 108 has a proximal opening (not shown in FIG. 1) and the distal opening 109 shown in FIG. 1. An interim fluid channel (not shown in FIG. 1) is formed in the body 108 between the proximal opening of the body 108 and the distal opening 109 of the body 108, as described herein. It should be appreciated that, in one example, the interim fluid channel aligns with at least one channel or lumen of the tubular member 104 (e.g., catheter). FIG. 2A shows a fluid jet 210 that is emitted along an axis (e.g., a longitudinal axis) from the distal opening 109 of the body 108. FIG. 2A also shows a direction of fluid flow along the axis, at reference numeral 211. The proximal opening of the body 108 is configured to interface with, and receive fluid from, a fluid delivery device (e.g., the fluid delivery mechanism described in connection with FIG. 1) that is internal and/or external to the medical device 102. The distal opening 109 of the body 108 is configured to emit fluid delivered from the fluid delivery device. An interim fluid channel (not shown in FIG. 2A) is formed in the body 108 between the proximal opening of the body 108 and the distal opening 109 of the body 108, as described herein. In one example, the fluid delivery device may be a fluid lumen or channel disposed within the tubular member 104 of the medical device 102, such that fluid (e.g., water or a saline solution) is delivered from a source at the proximal end of the medical device 102 to the proximal opening of the body 108 for egress through the distal opening 109 of the body 108. In this example, the body 108 is configured to deliver the fluid jet 210 as the fluid is emitted from the fluid delivery device.

The fluid jet 210 may be a fluid jet of water, saline, or other liquid that is delivered at a fluid pressure sufficient for tissue resection. For example, the fluid jet 210 may be emitted through the distal opening 109 of the body 108 at a fluid pressure of up to 60 atmospheric bars ("bars"), or approximately up to 870 pounds per square inch (psi). In one embodiment, the fluid jet 210 is emitted at a fluid pressure of 250 psi or less when the diameter of the distal opening 109 of the body 108 is 1 millimeters (mm). It should be appreciated that the proper fluid pressure may vary depending on system and device parameters, including but not limited to the tissue type, the fluid used for the fluid jet 210, the diameter of the distal opening 109 of the body 108, etc. In one example, the diameter of the distal opening 109 varies based on the channels of the tubular member 104 and the desired size of the intended area of tissue impact of the fluid jet 210. For example, a fluid pressure of between about 20-60 bars may be used for tissue resection, with, e.g., relatively lower pressures providing cleaner and more precise tissue piercings or perforations to minimize risk. In one example, when the distal opening 109 has a diameter of about 0.04 inches, less than about 250 psi of fluid pressure may be sufficient to pierce tissue (e.g., muscle tissue, diseased tissue, or other types of tissue to be treated by the medical device 102), and when the distal opening 109 has a diameter of about 0.05 inches, less than about 100 psi of fluid pressure may be sufficient to pierce tissue.. Higher fluid pressures may be desirable for tissue resection of mucosal and/or submucosal layers as opposed to fluid pressures for muscle tissue since the mucosal and submucosal layers of the GI tract may be tougher than muscle. In one example, fluid pressure of about 600 psi may pierce mucosal and/or submucosal tissue. The fluid pressure may also vary based on the type of distal opening 109 (e.g., the internal shape and the geometry of the distal opening 109). In one example, the distal opening 109 may be chamfered to disperse pressure, or conical/tapered inward (tapered from a proximal to distal direction) to focus the stream of fluid (e.g., fluid jet 210).

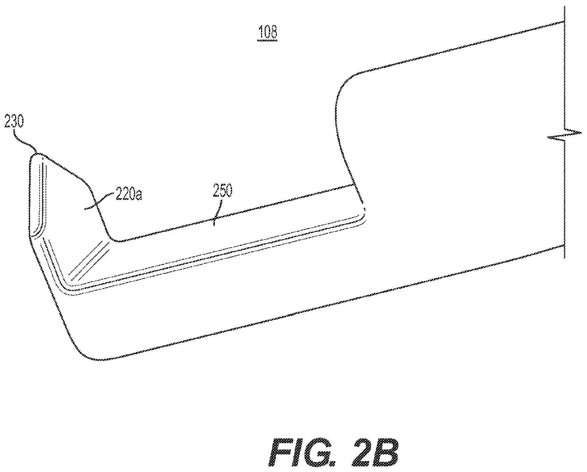

FIG. 2A also shows an outer surface of a distal wall 220. The distal wall 220 extends in a direction transverse to a longitudinal axis along which the fluid jet 210 is emitted. Referring to FIG. 2B, which shows a view of the body 108 according to an example embodiment, an inner, proximally-facing surface of the distal wall 220 is shown at reference numeral 220a. The inner surface 220a faces the distal opening 109 of the body 108. When fluid is delivered through the body 108, the fluid jet 210 is emitted from the distal opening 109 of the body 108 and the fluid jet 210 is received at the inner surface 220a of the distal wall 220. FIG. 2B also shows the distal wall 220 having a protrusion 230 at a top end of the distal wall 220 and extending in the direction transverse to the longitudinal axis at which the fluid jet 210 is emitted. The protrusion 230 may be a relatively sharp tip of the distal wall 220 that points in a radially outward direction. The protrusion 230 is configured to engage tissue (e.g., to pierce the tissue surface and/or be placed on the tissue surface). For example, the protrusion 230 engages the tissue boundary 114, described in connection with FIG. 1, to guide or affix the body 108 to a region proximate a tissue of interest (e.g., the target tissue 116) for ultimate performance of the fluid powered tissue resection methods described herein. In one example, the protrusion 230 is a hook or a hook-like feature that is configured to engage and affix to tissue. The protrusion 230 may comprise one or more tips or prongs.

As the fluid jet 210 is emitted at a sufficiently high fluid pressure to resect tissue, there is a desire to minimize or mitigate unintended tissue perforation. In one example, unmitigated fluid flow without a barrier to block the fluid jet 210 could quickly perforate organs/tissue not intended for tissue treatment. The protrusion 230 may prevent or limit unintended tissue perforation by blocking fluid flow past the tissue that is acquired by the protrusion 230. The protrusion 230 provides a solid surface (e.g., the inner surface 220a of the distal wall 220) for the fluid jet 210 to impact, which may dissipate the force of the fluid jet 210. As the fluid jet 210 impacts the solid surface, the fluid may "splash back." In order to mitigate or prevent unintended tissue perforation from the fluid splash back, the fluid splash back may be at a sufficiently low energy not to cause any damage to the surrounding healthy tissue (and, e.g., to avoid obscuring a camera view of operation). Thus, the protrusion 230 may be shaped to minimize the fluid energy of the splash back as the fluid jet 210 impacts the protrusion 230. For example, the solid surface (e.g., the inner surface 220(a) of the distal wall 220) may be flat, convex, or concave relative to the flow of the fluid jet 210. In general, the profiles of the distal wall 220 and the protrusion 230 may be optimized in terms of distance from the distal opening 109, shape, material, and thickness in order to direct the splash back safely. Referring back to FIG. 2A, this dissipation is shown at reference numeral 240, as fluid dissipates along the edges and sides of the body 108 and the distal wall 220 and causes minimal or no unintended tissue perforation.

FIG. 2B also shows a bed region ("bed") 250 of the body 108. The bed 250 in one example is a surface disposed along the longitudinal axis between the distal opening 109 of the body 108 and the distal wall 220 of the body 108. The bed 250 may define a space in the body 108 for receiving tissue between the distal opening 109 and the distal wall 220. In one example, the bed 250 is a surface that connects the inner surface 220a of the distal wall 220 to a side of the body 108 with the distal opening 109 of the body 108. The bed 250 may be used during a medical procedure to remove a resected tissue from a patient's body. For example, after the target tissue 116 is treated with the fluid-powered resection methods, the body 108 may be maneuvered such that the target tissue 116 is placed on the bed 250 and removed from the patient's body as the medical device 102 is removed. To assist in capturing the resected tissue and retaining it during retraction of the medical device 102 from the patient, the surface of bed 250 may be treated with a tacky coating or otherwise treated so that the resected tissue adheres to the surface of the bed 250.

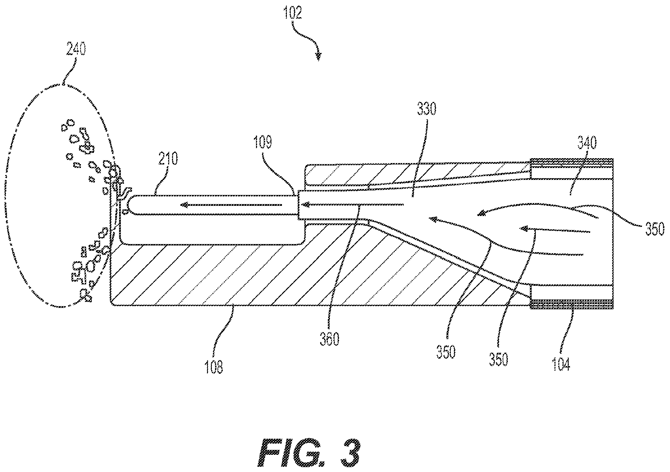

Reference is now made to FIG. 3, which shows a cross-sectional view of the medical device 102. FIG. 3 shows the cross-sectional view of the body 108 and a distal end of the tubular member 104 of the medical device 102. FIG. 3 shows an interim fluid channel 330 in the body 108 and a primary fluid channel 340 in the tubular member 104. As described in connection with FIG. 2A, the interim fluid channel 330 is formed between a proximal opening in the body 108 and the distal opening 109 of the body 108. In FIG. 3, fluid in the primary fluid channel 340 flows to the interim fluid channel 330, as shown by arrows 350, via the proximal opening of the body 108 (not shown in FIG. 3). In other words, the proximal opening of the body 108 interfaces with a distal opening of the primary fluid channel 340 such that fluid may flow between the primary fluid channel 340 and the interim fluid channel 330. The interim fluid channel 330 tapers from a proximal end to the distal end. In other words, the cross-sectional area and/or the diameter of the interim fluid channel 330 decreases from its proximal end to its distal end. As a result, as fluid flows through the interim fluid channel 330 in the distal direction (shown at arrow 360), fluid pressure increases. Thus, the fluid flows at a higher pressure toward the distal end of the interim fluid channel 330 when compared to the fluid flow at the proximal end of the interim fluid channel 330 and when compared to the fluid flow in the primary fluid channel 340. As a result, as the fluid jet 210 egresses from the interim fluid channel 330 at the distal opening 109 of the body 108, the fluid jet 210 is emitted at a higher fluid pressure relative to the fluid pressure of the primary fluid channel 340. In this example, the distal opening 109 of the body 108 operates as a nozzle to emit the fluid jet 210 at a high relative fluid pressure. As described herein, the fluid pressure of the fluid jet 210 is sufficiently high to perform tissue resection operations. Splash back is limited, as shown by the water dissipation at reference 240.

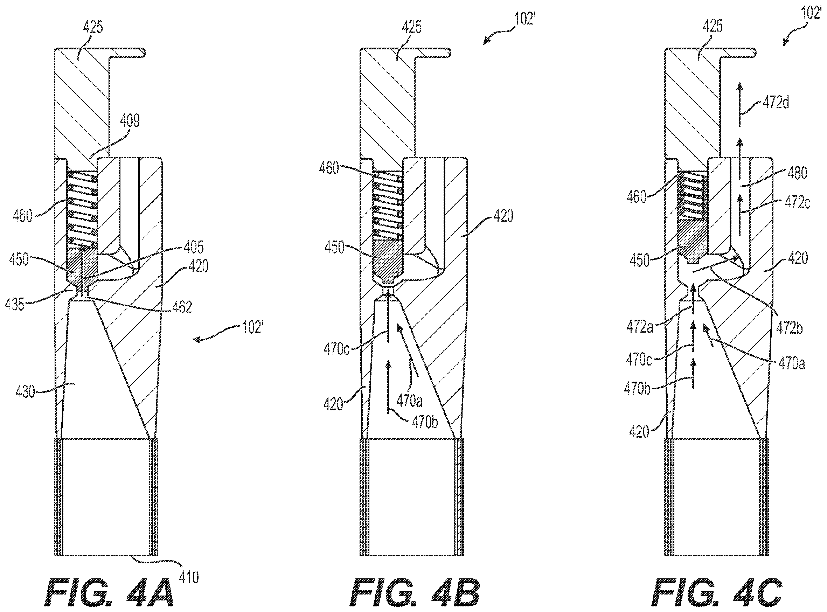

Reference is now made to FIGs. 4A-4C, which show a cross-sectional view of another embodiment a medical device 102'. FIG. 4A shows a tubular member 410, a distal end structure 420, and a body 425. The tubular member 410 may have the structure and functions of the tubular member 104, and the body 425 may have the structure and functions of the body 108. The distal end structure 420 may have the structure and functions of the distal end 106. The distal end structure 420 further includes a valve 450, and a spring 460. In the example depicted in FIG. 4A, fluid may be delivered to a distal end of the medical device 102'. At the distal end, after the fluid has traveled through the tubular member 410 to an interim fluid channel, shown at 430, the interim fluid channel 430 holds the fluid as the interim fluid channel 430 narrows.

The valve 450 is located in a distal portion of the interim fluid channel 430, distal to a narrowing 435 of the interim fluid channel 430. The narrowing 435 has a smaller diameter and/or cross-sectional area relative to portions of channel 430 distal to and proximal to the narrowing 435. The valve 450 is not fixed to the interim fluid channel 430 and is therefore able to translate longitudinally within channel 430, e.g., along a direction shown by arrow 405. The spring 460 also is located in the distal portion of the interim fluid channel 430, distal to the valve 450. The spring 460 is affixed to the valve 450 at a proximal end of the spring 460.

The spring 460 and the valve 450 are positioned between a proximal opening of the interim fluid channel 430, shown at reference 462, and a proximal end of the body 425. In one example, the body 425 is bonded to the distal end structure 420 such that the spring 460 is affixed to a surface of the body 425 at a distal end of the spring 460. In one example, the spring 460 is a coil spring that is compressible upon application of pressure (e.g., upon application of fluid pressure along the direction 405 on the valve 450). In one example, the valve 450 is a one-way valve that allows fluid to egress from the interim fluid channel 430 in a single direction.

Reference is now made to FIGs. 4B and 4C. FIG. 4B shows a closed state configuration of the valve 450. In FIG. 4B, arrows 470a-470c represent fluid flowing into the interim fluid channel 430. The fluid pressure increases at arrow 470c as the interim fluid channel 430 narrows toward the valve 450. In FIG. 4B, the valve is closed, and thus fluid does not egress from the interim fluid channel 430. FIG. 4C shows an open state configuration of the valve 450. In FIG. 4C, once the fluid reaches sufficient pressure (e.g., a threshold pressure), the proximal force exerted by the spring 460 on the valve 450 is overcome, and the pressure forces the valve 450 in a distal direction toward the spring 460 (e.g., along direction 405), thus compressing the spring 460 in the distal direction. Once the distal motion begins, a shape of the valve 450 may result in a greater area of the valve 450 exposed to fluid flow, forcing it to open quickly. Thus, when the spring 460 is in the compressed state (e.g., when the valve 450 and the spring 460 have moved in a distal direction beyond a threshold distance resulting from the threshold pressure), fluid may be able to flow around the valve, as shown in arrows 472a-472d, into an egress channel, shown at reference 480. Egress channel 480 may extend from the proximal opening 462 to the distal end structure 420, such that fluid may flow towards the body 425 e.g., in the direction of arrows 472a-472d. The fluid leaves the egress channel 480 and flows toward the protrusion (e.g., protrusion 230). As fluid pressure in the interim fluid channel 430 decreases (e.g., when the fluid supplied to the device 102' is decreased), the proximal pressure of the spring 460 on the valve 450 may be greater than the fluid pressure exerted on the valve 450, and the valve 450 may recede (e.g., travel in a proximal direction) to the closed state shown in FIG. 4B.

In one example, the fluid is emitted from the interim fluid channel 430 at a constant or relatively constant pressure. Thus, the mechanism described in FIGs. 4A-4C enables fluid to be emitted from the egress channel 480 at a constant or near constant pressure, which avoids a scenario where the fluid pressure gradually builds up during fluid egress from the interim fluid channel 430 and the distal opening 462. In some examples, it is advantageous for fluid to be emitted from the egress channel 480 at a substantially constant pressure to avoid unintended tissue damage during pressure build up or suboptimal tissue resection.

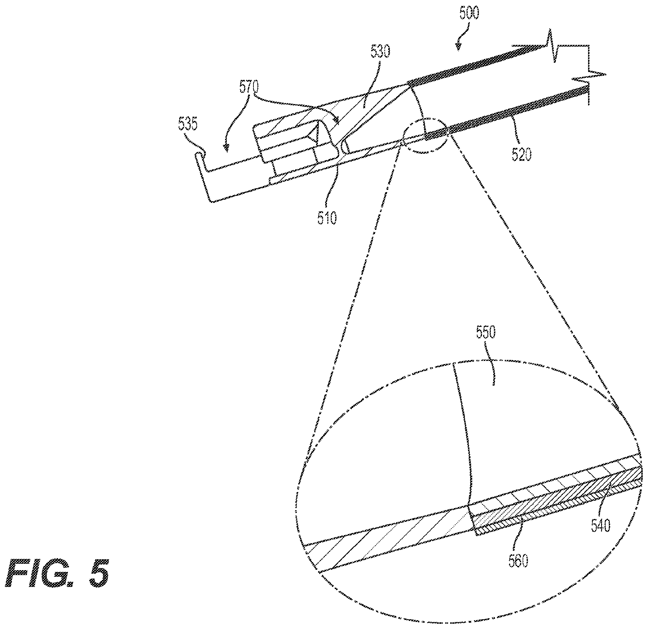

Reference is now made to FIG. 5. FIG. 5 shows a cross-sectional view of yet another embodiment of a medical device at 500. In general, the device 500 is configured for fluid powered tissue dissection and RF energy delivery to tissue. FIG. 5 shows the cross-sectional view of tubular member 520 and body 530. The tubular member 520 and the body 530 can have any of the structure and functions of tubular member 104, 420 and the body 108, 410, respectively. The medical device 500 is advantageous for endoscopic procedures to provide coagulative functions with RF delivery in addition to the tissue resection functions performed by the fluid powered system. The body 530 may be bonded or otherwise affixed to the tubular member 520. The body 530 is made of metal or other electrically conductive material. The body 530 is configured to conduct RF energy. For example, RF energy may be delivered to the body 530 via a conductive tube, wire, cable, or braid of the medical device 500. A conductive tube is shown at reference numeral 540 in the expanded view of the junction between the body 530 and the tubular member 520. The conductive tube 540 may be surrounded by insulating material, shown at reference numerals 550 (an inner insulation) and 560 (an outer insulation). Thus, the conductive tube 540 forms an electrical conduit to carry the RF energy to the body 530 and ultimately to distal wall 535 to coagulate tissue during a medical operation. The RF active components of the body 530 therefore includes the distal wall 535. Thus, in one example, the distal wall 535 may operate as the point of contact to the tissue to deliver RF energy for coagulation.

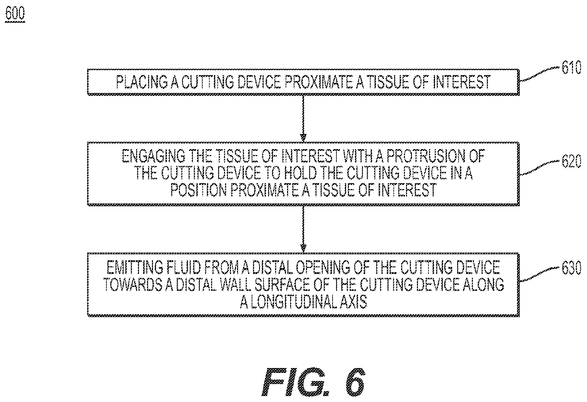

Reference is now made to FIG. 6, which shows an example flow chart 600 depicting operations for performing the fluid-powered tissue resection techniques, described herein. At operation 610, a resection device is placed proximate a tissue of interest. The resection device may be any of the medical devices 102, 102', 500 described herein. At operation 620, the tissue of interest is engaged with a protrusion of the resection device to hold the resection device in a position at the tissue of interest. At operation 630, fluid is emitted from a distal opening of the resection device towards a distal wall surface of the resection device along a longitudinal axis. The fluid resects the tissue of interest.

Other embodiments of the disclosure will be apparent to those skilled in the art from consideration of the specification and practice of the invention disclosed herein. It is intended that the specification and examples be considered as exemplary only, with a true scope and spirit of the invention being indicated by the following claims.

It should be understood that one or more of the aspects of any of the medical devices described herein may be using in combination with any other medical device known in the art, such as medical imaging systems or other scopes such as colonoscopes, bronchoscopes, ureteroscopes, duodenoscopes, etc., or other types of imagers.

It also should also be understood that one or more aspects of any of the medical devices described herein may be used for resection, cutting, or otherwise dissecting tissue in any part of the human body. For example, any of the medical devices described herein may be used in medical procedures where removal and/or detection of tissue is needed.

While principles of the present disclosure are described herein with reference to illustrative examples for particular applications, it should be understood that the disclosure is not limited thereto. Those having ordinary skill in the art and access to the teachings provided herein will recognize additional modifications, applications, and substitution of equivalents all fall within the scope of the examples described herein. Accordingly, the invention is not to be considered as limited by the foregoing description.

The following aspects are preferred embodiments of the invention.
1. A medical device comprising:
   a body having a proximal end with a proximal opening, the body defining a channel from the proximal opening to a distal opening configured to emit a fluid jet along a longitudinal axis of the body;
   the body further including a distal wall surface having a surface extending in a direction transverse to the longitudinal axis and facing the distal opening to receive the fluid jet, the body defining a space between the distal wall surface and the distal opening, the distal wall including a protrusion configured to engage tissue.
2. The medical device of aspect 1, wherein the distal opening emits the fluid jet at a pressure to pierce the tissue.
3. The medical device of aspect 2, wherein the pressure is at or below 250 pounds per square inch.
4. The medical device of any of the preceding aspects, wherein the distal opening has a diameter of approximately 1 millimeter or less.
5. The medical device of aspect any of the preceding aspects, wherein the proximal opening has a diameter that is larger than a diameter of the distal opening.
6. The medical device of aspect 1 or 5, wherein the channel tapers in cross-sectional size from the distal opening to the proximal opening.
7. The medical device of any of the preceding aspects, wherein the protrusion comprises one or more pointed tips to engage the tissue.
8. The medical device of any of the preceding aspects, wherein the body receives a fluid at the proximal opening and further comprises a valve and a spring configured to maintain the fluid within the body until a pressure of the fluid is above a predetermined threshold.
9. The medical device of aspect 1 or 8, wherein the valve is fixedly coupled to the spring, the valve and the spring are positioned between the proximal opening and the distal opening, and the valve is proximal to the spring.
10. The medical device of aspects 1, 8 or 9, wherein the valve is a one way valve, and the spring is a coil spring having a distal end fixed within the body.
11. The medical device of any of the preceding aspects, wherein the body is electrically medical to deliver radio frequency (RF) energy to the tissue.
12. The medical device of aspect 1 or 11, wherein the RF energy delivered to the body is conductive to the distal wall surface.
13. The medical device of any of the preceding aspects, wherein the body further comprises a bottom surface disposed along the longitudinal axis between the proximal opening and the distal wall surface to define a region to engage tissue.
14. The medical device of any of the preceding aspects, further comprising a flexible tube coupled to the proximal end of the body, the flexible tube including a channel to deliver a fluid to the body.
15. The medical device of aspect 1 or 14, wherein the flexible tube includes an electrically conductive tube, wire, cable, or braid for delivery of radio frequency (RF) energy to the body.

## Claims

1. A medical device comprising:
a body having a proximal end with a proximal opening, the body defining a channel from the proximal opening to a distal opening configured to emit a fluid jet along a longitudinal axis of the body;
the body further including a distal wall extending in a direction transverse to the longitudinal axis and having an inner surface which faces the distal opening to receive the fluid jet, the body defining a space between the distal wall and the distal opening, the distal wall including a protrusion configured to engage tissue,
wherein the body receives a fluid at the proximal opening and further comprises a valve and a spring configured to maintain the fluid within the body until a pressure of the fluid is above a predetermined threshold.

2. The medical device according to claim 1,
wherein the valve is fixedly coupled to the spring,
wherein the valve and the spring are positioned between the proximal opening and the distal opening, and
wherein the valve is proximal to the spring.

3. The medical device according to claim 1 or claim 2,
wherein the valve is a one way valve, and the spring is a coil spring having a distal end fixed within the body.

4. The medical device according to any one of claims 1-3,
wherein the body further includes an interim fluid channel comprising a narrowing,
wherein the valve is located in a distal portion of the interim fluid channel, distal to the narrowing of the interim fluid channel, and
wherein the spring also is located in the distal portion of the interim fluid channel, distal to the valve.

5. The medical device of claim 4,
wherein, once the pressure of the fluid reaches the predetermined threshold, a proximal force exerted by the spring on the valve is overcome and the fluid pressure forces the valve in a distal direction toward the spring, thus compressing the spring in the distal direction, and
wherein, once the distal motion of the valve begins, a shape of the valve results in a greater area of the valve being exposed to fluid flow.

6. The medical device according to any one of the preceding claims 1-5,
further comprising a bed surface disposed along the longitudinal axis between the distal opening of the body and the distal wall of the body, the bed surface being configured to be used during a medical procedure to remove a resected tissue from a patient's body.

7. The medical device according to any one of the preceding claims 1-6,
wherein the medical device further comprises a tubular member, preferably a catheter; and
wherein the body is disposed on a distal end of the tubular member.

8. The medical device according to any one of the preceding claims 1-7, wherein the distal opening emits the fluid jet at a pressure to pierce the tissue.

9. The medical device according to claim 8, wherein the pressure is at or below 250 pounds per square inch.

10. The medical device according to any one of the preceding claims 1-9, wherein the distal opening has a diameter of approximately 1 millimeter or less.

11. The medical device according to any one of the preceding claims 1-10, wherein the proximal opening has a diameter that is larger than a diameter of the distal opening.

12. The medical device according to any one of the preceding claims 1-11, wherein the channel tapers in cross-sectional size from the proximal opening to the distal opening.

13. The medical device according to any one of the preceding claims 1-12, wherein the protrusion comprises one or more pointed tips to engage the tissue.

14. The medical device according to any one of the preceding claims 1-13, wherein the body is electrically conductive to deliver radio frequency, RF, energy to the tissue.

15. The medical device of claim 14, wherein the RF energy delivered to the body is conductive to the distal wall surface.
